# EUROPÄISCHE PATENTSCHRIFT

(11) **EP 2 621 373 B1**
(45) Veröffentlichungstag und Bekanntmachung des Hinweises auf die Patenterteilung: **09.07.2014**
(21) Anmeldenummer: 11773860.9
(22) Anmeldetag: 13.09.2011
(51) Int. Cl.: A61B 17/16, A61B 17/32, A61B 17/00, A61B 17/115

(54) **KUPPLUNGSTEIL ZU EINEM CHIRURGISCHEN HANDSTÜCK**
COUPLING PART FOR A SURGICAL HANDPIECE
PARTIE D'ACCOUPLEMENT POUR UNE PIÈCE À MAIN CHIRURGICALE

(30) Priorität: 29.09.2010 CH 15852010
(43) Veröffentlichungstag der Anmeldung: 07.08.2013
(73) Patentinhaber: Da Rold, Orlando, 4500 Solothurn (CH)
(72) Erfinder: DA ROLD, Orlando, CH-4500 Solothurn (CH); DECK, René, CH-4532 Feldbrunnen (CH)
(74) Vertreter: Fleck, Hermann-Josef
(86) Internationale Anmeldenummer: PCT/IB2011/054003
(87) Internationale Veröffentlichungsnummer: WO 2012/042417

(56) Entgegenhaltungen:
- EP-A2- 1 845 298
- EP-B1- 0 866 674
- US-A1- 2004 092 991
- US-A1- 2007 145 742

## Beschreibung

Die vorliegende Erfindung betrifft ein Kupplungsteil zu einem chirurgischen Handstück gemäss Oberbegriff des Patentanspruchs 1.

Bestimmte chirurgische Geräte und Systeme bestehen aus einem motorisch angetriebenen Handstück das eine Vielfalt chirurgischer Vorrichtungen antreibt. In EP1773215 ist z.B. ein Schneidmesser beschrieben wie es für die Arthroskopie verwendet wird. Solche Vorrichtungen werden mit einem Kupplungsteil versehen, das in der dafür vorgesehenen Bohrung eingeführt wird, wobei damit gewisse Teile der Vorrichtung mit dem Antriebsteil des Handstückes formschlüssig verbunden werden und das Kupplungsteil im Handstück einklinkt.

Eine typische Vorrichtung dieser Art ist in der EP0866674B1 (Basis für den Oberbegriff des Anspruchs 1) vorgestellt. Diese realisiert das Einklinken mit einer einfachen Klinkenstruktur die seit langer Zeit bekannt ist und vielfach in den verschiedensten Gebieten der Technik eingesetzt wird. Der grosse Nachteil solcher Klinkenstrukturen liegt darin, dass für eine sichere Verankerung des zu haltenden Teiles der Haken an einer dafür vorgesehenen Fläche oder Kante vollständig und sicher einhaken muss.

Bei den in der Chirurgie verwendeten angetriebenen Handstücken handelt es sich meist um relativ feine Geräte, welche aber sicher bedienbar sein müssen. Der Operateur wird das Handstück, insbesondere die Klinkenstruktur mit Gummihandschuhen bedienen müssen und das Gerät muss trotz filigraner Konstruktion, absolut sicher funktionieren. Die einfache und sichere Bedienung muss gewährleistet sein, wenn der Chirurg selbst, ein Assistent, die OP-Schwester oder irgend Jemand im Operationssaal das Gerät bedient. In dieser Schrift ist mit "Operateur" eine Person benannt, die das Gerät bedient.

Zur sicheren Funktion gehört nicht nur das sichere Einrasten der Klinkenstruktur. Der Operateur muss auch ein klar verständliches und bestätigendes Signal erhalten, dass die Klinke eingerastet ist. Ein solches Signal kann akustisch oder mechanisch erfolgen. Das akustische Signal mag im Operationssaal im Lärm der andern Geräte leicht untergehen, weshalb wünschbar ist, dass beim Einrasten des Kupplungsteiles in das Handstück zusätzlich noch ein deutliches fühlbares, mechanisches Zeichen an den mit Handschuhen behinderten Fingern erfolgt, welches zeigt "jetzt ist eingerastet und sicher".

Die in EP0866674B1 vorgestellte Klinkenstruktur zeigt in der Praxis offensichtliche Nachteile. Die feine Klinke meist aus Kunststoff gefertigt gibt beim Einklinken ein kleines "klick" als akustisches Signal. Wenn dieses Signal nicht hör- oder fühlbar ist, weiss man nicht mit der notwendigen Sicherheit, ob der Kupplungsteil sicher eingeklinkt hat. Mit den heute verwendeten Vorrichtungen ist dies nicht gewährleistet.

Die vorliegende Erfindung stellt sich nunmehr die Aufgabe ein Kupplungsteil eingangs genannten Art derart zu verbessern, dass die Vorteile der bekannten Klinkenstruktur erhalten bleiben, das Gerät aber in der dafür vorgesehenen Position absolut sicher einklinkt, sich nach Möglichkeit noch zusätzlich verklemmt und dem Chirurgen und dem OP-Personal auf jeden Fall nebst dem akustischen auch noch ein fühlbares mechanisches Signal gibt, dass sicher eingeklinkt ist.

Diese Aufgabe löst eine Vorrichtung mit den Merkmalen des Patentanspruches 1. Weitere erfindungsgemässe Merkmale gehen aus den abhängigen Ansprüchen hervor und deren Vorteile sind in der nachfolgenden Beschreibung erläutert.

In der Zeichnung zeigt:
- Fig 1: Handstück mit Bohrung
- Fig 2: Detail Handstück mit Bohrung
- Fig 3: Kupplungsteil mit Schieber in distaler Lage
- Fig 4: Kupplungsteil mit Schieber in proximaler Lage
- Fig 5: Kupplungsteil mit Schieber in distaler Lage eingebaut im Handstück
- Fig 6: Kupplungsteil mit Schieber in proximaler Lage eingebaut im Handstück
- Fig 7: Schieber

Die Figuren stellen mögliche Ausführungsbeispiele dar, welche in der nachfolgenden Beschreibung erläutert werden. In allen Figuren ist links die proximale, die in der Anwendung dem Körper zugewandte Seite und rechts die distale, dem Körper abgewandte Seite dargestellt.

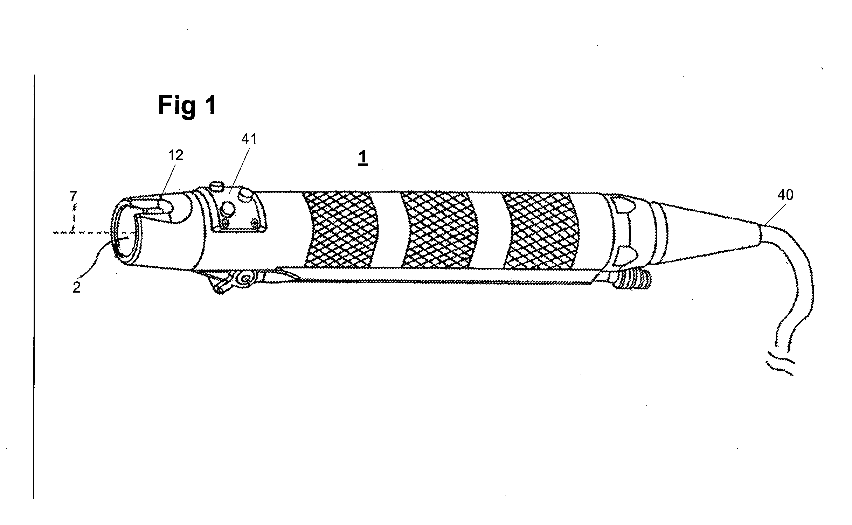

Fig 1 zeigt ein motorisch angetriebenes Handstück 1. Solche Handstücke sind Teil einer Antriebseinheit und werden zum Antrieb und zur Halterung einer Vielfalt chirurgischer Vorrichtungen verwendet. Distal ist die Zuführung 40 des Antriebsmediums oder der mechanischen Antriebsteile angeordnet und proximal befindet sich die Bohrung 2 zur Aufnahme chirurgischer Vorrichtungen. Das in Fig 1 dargestellte zeigt Handstück 1 beispielhaft eine Bedienungszone 41. Mit den darin befindlichen Knöpfen oder Schaltern kann der Operateur im vorliegenden und in Fig 1 dargestellten Fall den Antrieb mittels Knöpfen 41 bedienen.

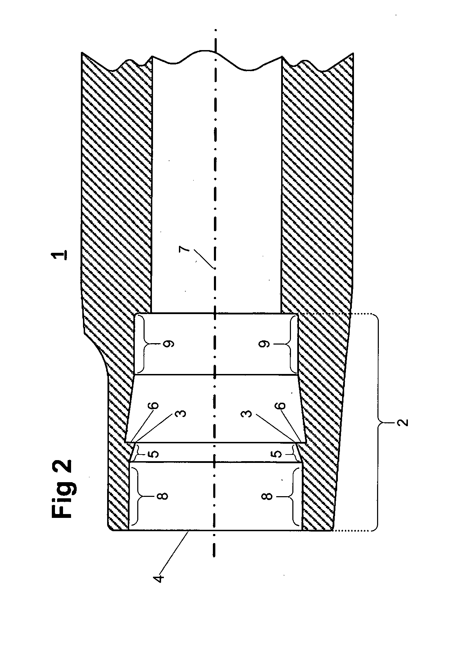

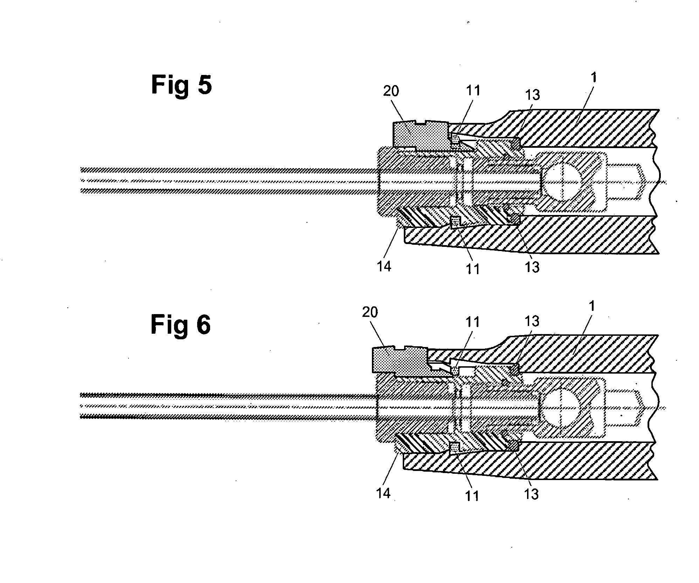

Die Bohrung 2 ist für die Führung der chirurgischen Vorrichtung, im vorliegenden Fall für den Kupplungsteil 10 vorgesehen. Der Kupplungsteil 10 wird in die Bohrung formführend eingeführt und gehalten. Die Bohrung 2 hat zwei Aufgaben. Sie nimmt den Kupplungsteil 10 wie Fig 6 zeigt in der Art auf, das Handstück 1 und Kupplung 10 fest miteinander verbunden sind. Dazu weist die Bohrung 1 (Fig 2) zwei Führungen auf. Eine proximale Führungszone 8 und eine distale Führungszone 9, wobei der Durchmesser der proximalen Führung 8 grösser ist als der Durchmesser der distalen Führung 9.

Um den Kupplungsteil 10 in der Bohrung 2 des Handstückes zu sichern, ist die Bohrung 2 mit einem Bund 3 versehen (Fig 2). Dieser Bund 3 bildet auf der proximalen Seite einen Anzug 5, damit der Kupplungsteil 10 geführt in die Bohrung 2 eingebracht werden kann. Auf der distalen Seite, dem Hauptteil des Handstückes 1 zugewandt befindet sich eine zur Hauptachse 7 senkrecht verlaufende Andrehung 6. Der Durchmesser des Bundes 3 ist gleich oder grösser dem Durchmesser der distalen Führung 9. Am Kupplungsteil 10 sind zwei Ringe 11,13 angebracht. Der O-Ring 13 dient der Abdichtung und Führung des Kupplungsteiles 10 in der distalen Führung 9 der Bohrung, während der Sicherungsring 11 der Führung und der Sicherung dient. Die vordere Führung des Kupplungsteiles 10 im Handstück 1 wird durch den Körper 14 des Kupplungsteiles 10 (Fig 5, 6) in der proximalen Führung 8 (Fig 2) sicher gestellt.

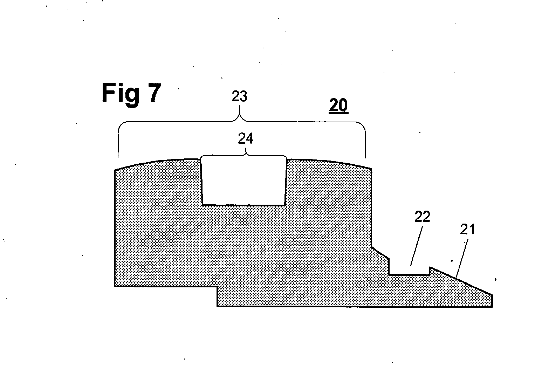

Die Sicherung der Kupplungsteiles 10 im Handstück 1 erfolgt nach dem Einführen des Kupplungsteiles 10 durch den Sicherungsring 11. Zum einen wird beim Einführen des Kupplungsteiles 10 in die Bohrung 2 des Handstücks 1 der im Durchmesser leicht grössere, aber elastische Sicherungsring 11 mit einem für den Operateur deutlich spürbaren Widerstand und einem ersten klick über den Bund 3 geschoben. Der Operateur weiss nun: "Der Kupplungsteil 10 befindet sich nun im Handstück 1 an der richtigen Stelle". Nun wird mittels Schieber 20, der Sicherungsring 11 an einer Stelle erweitert, so dass der Sicherungsring 11 an der Andrehung 6 in der Bohrung 2 des Handstücks 1 ansteht (Fig 5). Für die Führung des Schiebers 20 ist im Kupplungsteil 10 eine Aussparung 12 vorgesehen, in der derselbe um einige Millimeter verschoben werden kann (Fig 1,3,4 und 5,6). Der Schieber 20 ist distal mit einem Anzug 21 versehen (Fig 7), der in einer Quernut 22 endet.

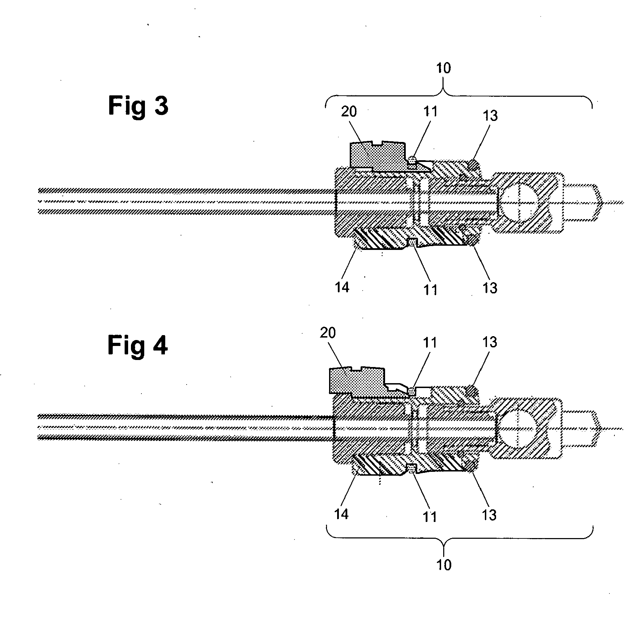

Beim Einführen des Kupplungsteiles 10 in die Bohrung 2 des Handstücks 1 befindet sich der Schieber 20 auf der proximalen Seite des Kupplungsteiles 10. Im Handstück 1 ist für den Schieber 20 eine Aussparung 12 (Fig 1) vorgesehen. Wird der Kupplungsteil 10 dann in die Bohrung 2 des Handstücks 1 eingeschoben, wird der Schieber 20 (Fig 4 und 6) in proximaler Lage, den Sicherungsring 11 nicht oder nur wenig berühren. Um das Kupplungsteil 10 im Handstück 1 zu sichern wird dann der Schieber 20 in distaler Richtung verschoben.

Durch die distale Verschiebung des Schiebers 20 wird der Sicherungsring 11 durch den Anzug 21 am Schieber 20 vergrössert, bis er sich in der Quernut 22 befindet. In der distalen Endstellung des Schiebers 20 klickt der Sicherungsring 11 in die Quernut 22 des Schiebers 20 ein (Fig 3 und 5). Im Bereich des Schiebers 20 hat der Sicherungsring 11 an dieser Stelle auch im eingeklickten Zustand radial eine grössere Distanz von der Hauptachse 7, so dass er hinter dem Bund 3 an der Andrehung 6 ansteht und so den Kupplungsteil 10 gegen unbeabsichtigtes Herausziehen sichert. Der Sicherungsring kann aus Gummi (z.B. O-Ring), Kunststoff oder Metall hergestellt sein. Auch der Querschnitt, in den Zeichnungen rund dargestellt, kann alle möglichen Formen haben.

Wenn als Sicherungsring 11 ein aus elastischem Kunststoff oder Gummi hergestellter Ring eingesetzt wird, muss der Schieber 20 nicht in proximaler Position sein, um das Kupplungsteil 10 im Handstück 1 zu sichern. Wenn in diesem Fall der Sicherungsring 11 durch den in distaler Position befindlichen Schieber 20 bereits radial erweitert ist, wird er über den Anzug 5 und den Bund 3 gedrückt und steht dann trotzdem sicher an der Andrehung 6 an. Das Kupplungsteil 10 hält also sicher im Handstück 1.

Um das Kupplungsteil 10 wieder aus dem Handstück 1 zu entfernen, wird der Schieber 20 zuerst in proximaler Richtung verschoben. Erst wenn der im Durchmesser elastische Sicherungsring 11 wieder den ursprünglichen Durchmesser erreicht hat, also radial überall im Abstand zur Hauptachse 7 die gleiche Distanz aufweist, kann das Kupplungsteil 10 aus dem Handstück 1 entfernt werden. Auch dieses Entfernen muss bewusst erfolgen, weil der Sicherungsring 11 über den Bund 3 gezogen werden muss.

Damit der Operateur, der normalerweise mit Gummihandschuhen arbeitet den Schieber 20 sicher bedienen kann, ist dieser mit einen gegen den Aussendurchmesser des Kupplungsteils 10 hin mit einem für die Finger spürbaren Griffteil 23 versehen (Fig 7). Der Griffteil 23 weist mindestes eine Kerbe 24 auf, damit der Operateur auch mit Gummihandschuhen und dadurch verminderter Tastfähigkeit der Finger die Stellung und vor allem auch das sichere Einklicken des Sicherungsrings 11 in die Quernut 22 gut spürt.

## Patentansprüche

1. Vorrichtung mit einem chirurgischen Handstück (1) und einem Kupplungsteil (10), das in eine Bohrung (2) des Handstücks (1) einführbar ist und formschlüssig in die Bohrung (2) passt, wobei die Bohrung (2) einen Bund (3) aufweist, welcher proximal einen Anzug (5) und distal eine zur Hauptachse (7) des Handstücks (1) in einem Winkel von 85° bis 90° verlaufende, flache Andrehung (6) aufweist,
**dadurch gekennzeichnet,**
**dass** das Kupplungsteil (1) einen flexiblen Sicherungsring (11) aufweist und einen in der Längsrichtung parallel zur Mittelachse des Kupplungsteiles (1) proximal /distal verschiebbaren Schieber (20) aufweist,
wobei der Schieber (20) den Sicherungsring (11) an einer Stelle erweitert, so dass der Sicherungsring (11) an der Andrehung (6) in der Bohrung (2) des Handstücks (1) ansteht.

2. Vorrichtung gemäß Anspruch 1,
**dadurch gekennzeichnet,**
**dass** der Schieber (20) distal einen Anzug (21) aufweist, der proximal in einer Quernut (22) endet, wobei Anzug (21) und Quernut (22) der Hauptachse (7) abgewandt, angeordnet sind.

3. Vorrichtung gemäß Anspruch 1,
**dadurch gekennzeichnet,**
**dass** der Schieber einen ausgeprägten Griffteil (23) mit mindestens einer Kerbe (24) aufweist.

4. Vorrichtung gemäß Anspruch 1,
**dadurch gekennzeichnet,**
**dass** der Außendurchmesser des Sicherungsringes (11) radial größer ist als der Innendurchmesser des Bundes (3).

5. Vorrichtung gemäß Anspruch 1,
**dadurch gekennzeichnet,**
**dass** die Basis der Quernut (22) des Schiebers (20) radial weiter von der Hauptachse (7) entfernt ist, als der Innendurchmesser des Sicherungsringes (11).

6. Vorrichtung gemäß Anspruch 1,
**dadurch gekennzeichnet,**
**dass** der Sicherungsring (11) in proximaler Lage des Schiebers (20) in seinem Umfang in etwa rund ist.

7. Vorrichtung gemäß Anspruch 1,
**dadurch gekennzeichnet,**
**dass** der Sicherungsring (11) distaler Lage des Schiebers (20) in der Quernut (22) des Schiebers (20) liegt.

## Claims

1. Device with a surgical handpiece (1) and with a coupling part (10) that can be inserted into a bore (2) of the handpiece (1) and engages in the bore (2) with a form fit, wherein the bore (2) has a collar (3) which, at the proximal end, has a taper (5) and, at the distal end, has a flat turned portion (6) extending at an angle of 85° to 90° to the main axis (7) of the handpiece (1), **characterized in that** the coupling part (10) has a flexible securing ring (11) and has a slide (20) which is movable proximally/distally in the longitudinal direction parallel to the central axis of the coupling part (10), wherein the slide (20) widens the securing ring (11) at one location, such that the securing ring (11) rests on the turned portion (6) in the bore (2) of the handpiece (1).

2. Device according to Claim 1, **characterized in that** the slide (20) has, at the distal end, a taper (21) which ends proximally in a transverse groove (22), wherein taper (21) and transverse groove (22) are arranged facing away from the main axis (7).

3. Device according to Claim 1, **characterized in that** the slide has an embossed grip part (23) with at least one notch (24).

4. Device according to Claim 1, **characterized in that** the external diameter of the securing ring (11) is radially greater than the internal diameter of the collar (3).

5. Device according to Claim 1, **characterized in that** the base of the transverse groove (22) of the slide (20) is radially farther away from the main axis (7) than the internal diameter of the securing ring (11).

6. Device according to Claim 1, **characterized in that** the securing ring (11), in the proximal position of the slide (20), is approximately round in its circumference.

7. Device according to Claim 1, **characterized in that** the securing ring (11), in the distal position of the slide (20), lies in the transverse groove (22) of the slide (20).

## Revendications

1. Dispositif comprenant une pièce à main chirurgicale (1) et une partie d'accouplement (10) qui peut être introduite dans un alésage (2) de la pièce à main (1) et qui s'adapte par engagement positif dans l'alésage (2), l'alésage (2) présentant un épaulement (3) qui présente, en position proximale, une dépouille (5) et, en position distale, une portée plate (6) s'étendant suivant un angle de 85 ° à 90 ° par rapport à l'axe principal (7) de la pièce à main (1),
**caractérisé en ce que**
la partie d'accouplement (10) présente une bague de fixation flexible (11) et un coulisseau (20) déplaçable en sens proximal/distal dans la direction longitudinale parallèlement à l'axe médian de la partie d'accouplement (10),
le coulisseau (20) élargissant à un endroit la bague de fixation (11) de telle sorte que la bague de fixation (11) soit en affleurement avec la portée (6) dans l'alésage (2) de la pièce à main (1).

2. Dispositif selon la revendication 1,
**caractérisé en ce que**
le coulisseau (20) présente en position distale une dépouille (21) qui se termine en position proximale dans une rainure transversale (22), la dépouille (21) et la rainure transversale (22) étant disposées de manière détournée de l'axe principal (7).

3. Dispositif selon la revendication 1,
**caractérisé en ce que**
le coulisseau présente une partie de préhension en relief (23) avec au moins une encoche (24).

4. Dispositif selon la revendication 1,
**caractérisé en ce que**
le diamètre extérieur de la bague de fixation (11) est radialement supérieur au diamètre intérieur de l'épaulement (3).

5. Dispositif selon la revendication 1,
**caractérisé en ce que**
la base de la rainure transversale (22) du coulisseau (20) est plus éloignée radialement de l'axe principal (7) que le diamètre intérieur de la bague de fixation (11).

6. Dispositif selon la revendication 1,
**caractérisé en ce que**
la bague de fixation (11), dans la position proximale du coulisseau (20), a une périphérie approximativement circulaire.

7. Dispositif selon la revendication 1,
**caractérisé en ce que**
la bague de fixation (11), dans la position distale du coulisseau (20), est située dans la rainure transversale (22) du coulisseau (20).
